# EUROPEAN PATENT APPLICATION

(11) **EP 1 386 639 A1**
(43) Date of publication of application: **04.02.2004**
(21) Application number: 02078143.1
(22) Date of filing: 31.07.2002
(51) Int. Cl.: A61P 35/00, A61P 35/02, A61K 39/395, C07K 14/47

(54) **Use of 78 genes identified to be involved in tumor development for the development of anti-cancer drugs and diagnosis of cancer**

(71) Applicant: Kylix B.V., 3971 JD Driebergen (NL)
(72) Inventor: Lund, Anders Henrik, 1066 HT Amsterdam (NL); Berns, Antonius Jozef Maria, 2082 BD Santpoort-Zuid (NL); van Lohuizen, Maarten Matthijs Sharif, 1011 TT Amsterdam (NL); Martins, Carla Pedro, 1073 BC Amsterdam (NL); Mikkers, Henricus Martinus Maria, 1068 SL Amsterdam (NL); Lenz, Jack Richard, South Salem, NY 10590 (US)
(74) Representative: Van Someren, Petronella F. H. M.

(57) **Abstract**

The invention relates to the use of inhibitors of the expressed proteins of the murine genes and/or their human homologues listed in Table 1 for the preparation of a therapeutical composition for the treatment of cancer, in particular for the treatment of solid tumors of lung, colon, breast, prostate, ovarian, pancreas and leukemia.The invention also relates to the therapeutical compositions comprising the inhibitors and to methods for development of the inhibitor compounds.

SEQUENCE LISTING

<110> Kylix B.V.
Lund, Anders Hendrik
Berns, Antonius Jozef Maria
van Lohuizen, Maarten Matthijs Sharif
Martins, Carla Pedro
Mikkers, Henricus Martinus Maria
Lenz, Jack Richard

<120> Use of genes identified to be involved in tumor development for the
development of anti-cancer drugs

<130> L/AM16/AvK/7

<160> 10

<170> PatentIn version 3.1

<210> 1
<211> 60
<212> DNA
<213> Artificial

<400> 1
cgaagagtaa ccgttgctag gagagaccgt ggtgaatgag actggtgtcg acactagtgg 60

<210> 2
<211> 45
<212> DNA
<213> Artificial

<400> 2
gatccactag tgtgacacag tctctaattt ttttttttaa aaaaa 45

<210> 3
<211> 23
<212> DNA
<213> Artificial

<400> 3
gctagcttgc caaactcagg tgg 23

<210> 4
<211> 27
<212> DNA
<213> Artificial

<400> 4
cgaagagtaa cgttgctagg agagacc 27

<210> 5
<211> 24
<212> DNA
<213> Artificial

<400> 5

gccaaaccta caggtggggt cttt 24

<210> 6
<211> 25
<212> DNA
<213> Artificial

<400> 6
gtggctgaat gagactggtg tcgac 25

<210> 7
<211> 22
<212> DNA
<213> Artificial

<400> 7
atcggacaga cacagataag tt 22

<210> 8
<211> 24
<212> DNA
<213> Artificial

<400> 8
gccaaaccta caggtggggt cttt 24

<210> 9
<211> 22
<212> DNA
<213> Artificial

<400> 9
aaacctgtga tgcctgacca gt 22

<210> 10
<211> 24
<212> DNA
<213> Artificial

<400> 10
gcaactgaga cctgcaaagc ttgt 24

## Description

The present invention relates to the use of the murine genes identified by retroviral insertional tagging as well as their human homologues for the identification and development of anti-cancer drugs, like small molecule inhibitors, antibodies, antisense molecules, RNA interference (RNAi) molecules and gene therapies against these genes and/or their expression products, and especially anti-cancer drugs effective against solid tumors of e.g. lung, colon, breast, prostate, ovarian, pancreas and leukemia. The invention further relates to pharmaceutical preparations comprising one or more of said inhibitors and methods for the treatment of cancer using said pharmaceutical preparations.

After a quarter century of rapid advances, cancer research has generated a rich and complex body of knowledge revealing cancer to be a disease involving dynamic changes in the genome. Cancer is thought to result from at least six essential alterations in cell physiology that collectively dictate malignant growth: self-sufficiency in growth signals, insensitivity to growth-inhibitory (anti-growth) signals, evasion of programmed cell death (apoptosis), limitless replicative potential, sustained angiogenesis, and tissue invasion and metastasis.

In general, these essential alterations are the result of mutations in genes involved in controlling these cellular processes. These mutations include deletions, point mutations, inversions and amplifications. The mutations result in either an aberrant level, timing, and/or location of expression of the encoded protein or a change in function of the encoded protein. These alterations can affect cell physiology either directly, or indirectly, for example via signalling cascades.

Identifying genes which promote the transition from a normal cell into a malignant cell provides a powerful tool for the development of novel therapies for the treatment of cancer.

One of the most common therapies for the treatment of cancer is chemotherapy. The patient is treated with one or more drugs which function as inhibitors of cellular growth and which is thus intrinsically toxic. Since cancer cells are among the fastest growing cells in the body, these cells are severely affected by the drugs used. However, also normal cells are affected resulting in, besides toxicity, very severe side-effects like loss of fertility.

Another commonly used therapy to treat cancer is radiation therapy. Radiotherapy uses high energy rays to damage cancer cells and this damage subsequently induces cell cycle arrest. Cell cycle arrest will ultimately result in programmed cell death (apoptosis). However, also normal cells are irradiated and damaged. In addition, it is difficult to completely obliterate, using this therapy, all tumor cells. Importantly, very small tumors and developing metastases cannot be treated using this therapy. Moreover, irradiation can cause mutations in the cells surrounding the tumor which increases the risk of developing new tumors. Combinations of both therapies are frequently used and a subsequent accumulation of side-effects is observed.

The major disadvantage of both therapies is that they do not discriminate between normal and tumor cells. Furthermore, tumor cells have the tendency to become resistant to these therapies, especially to chemotherapy.

Therapies directed at tumor specific targets would increase the efficiency of the therapy due to i) a decrease in the chance of developing drug resistance, ii) the drugs used in these tumor specific therapies are used at much lower concentrations and are thus less toxic and iii) because only tumor cells are affected, the observed side-effects are reduced.

The use of tumor specific therapies is limited by the number of targets known. Since tumors mostly arise from different changes in the genome, their genotypes are variable although they may be classified as the same disease type. This is one of the main reasons why not a single therapy exists that is effective in all patients with a certain type of cancer. Diagnosis of the affected genes in a certain tumor type allows for the design of therapies comprising the use of specific anti-cancer drugs directed against the proteins encoded by these genes.

Presently, only a limited number of genes involved in tumor development are known and there is a clear need for the identification of novel genes involved in tumor development to be used to design tumor specific therapies and to define the genotype of a certain tumor.

In the research that led to the present invention, a number of genes were identified by proviral tagging to be involved in tumor development. Proviral tagging is a method that uses a retrovirus to infect normal vertebrate cells. After infection, the virus integrates into the genome thereby disrupting the local organization of the genome. This integration is random and, depending on the integration site, affects the expression or function of nearby genes. If a gene involved in tumor development is affected, the cell has a selective advantage to develop into a tumor as compared to the cells in which no genes involved in tumor development are affected. As a result, all cells within the tumor originating from this single cell will carry the same proviral integration. Through analysis of the region nearby the retroviral integration site, the affected gene can be identified. Due to the size of the genome and the total number of integration sites investigated in the present invention, a gene that is affected in two or more independent tumors must provide a selective advantage and therefore contribute to tumor development. Such sites of integration are designated as common insertion sites (CIS).

The genes claimed in the present invention are all identified by common insertion sites and are so far not reported to be involved in tumor development. The novel cancer genes that were identified in this manner are the following: *Cd6, Cd83, Ly108, Sdc4,* and *Selpl,* encoding cell-surface proteins; *Ggta1, Pla2g7, Rabggtb,* encoding enzymes; *Ak4, Camk2d, Camkk2, Dgke,* mouse homolog of *PSK, Nori2, Ntkl, Pim3,* and *Ptk91,* encoding kinases; mouse homolog *of DUSP5* and *Ptpn1,* encoding phosphatases; *Wisp2* and *Wnt5b,* encoding secreted factors; *Cabp2, Calm2, Corolc, Fbxw4, Fkbp10, Gnb1, Hbs11, Kif13a,* mouse homolog of *PRAX-1, Swap70,* and *Tiam2,* encoding signaling proteins; *Elf4, Gfi1b, Hivep1, Klf3, Maz, Mef2d, Supt4h, Zfhxlb,* and *Znfnla3,* encoding proteins involved in transcriptional regulation; *Cil-pending, Tsga2,* genes with the following Celera identification codes mCG10088, mCG10294, mCG14584, mCG15383, mCG16286, mCG16752, mCG16756, mCG19525, mCG20092, mCG21612, mCG2332, mCG49753, mCG50456, mCG5049, mCG55300, mCG55520, mCG55784, mCG57225, mCG57228, mCG57816, mCG59306, mCG59312, mCG60024, mCG60113, mCG60609, mCG61858, mCG62190, mCG62286, mCG62490, mCG63480, mCG65233, mCG7764, and mCG9361. These genes are also listed in Table 1.

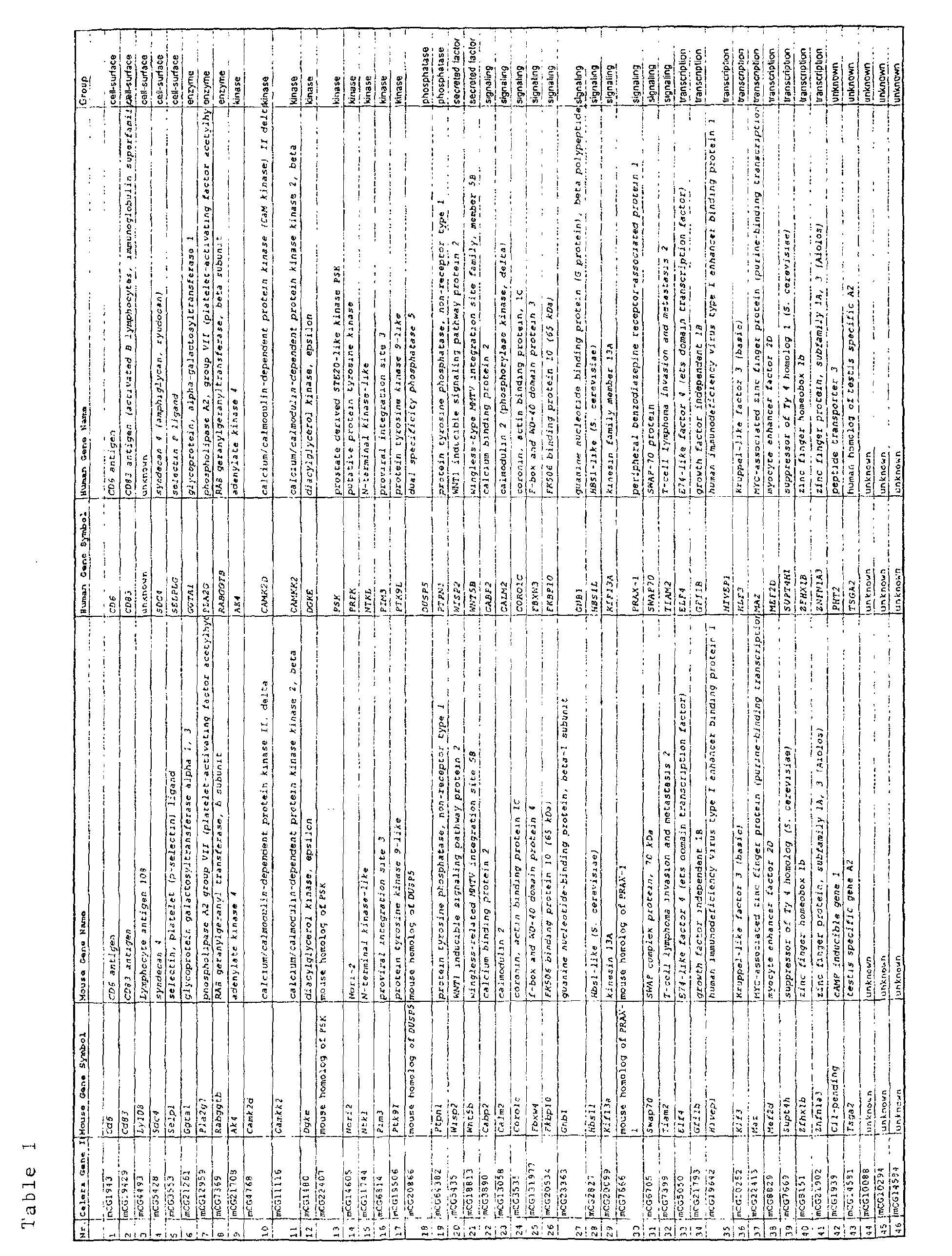

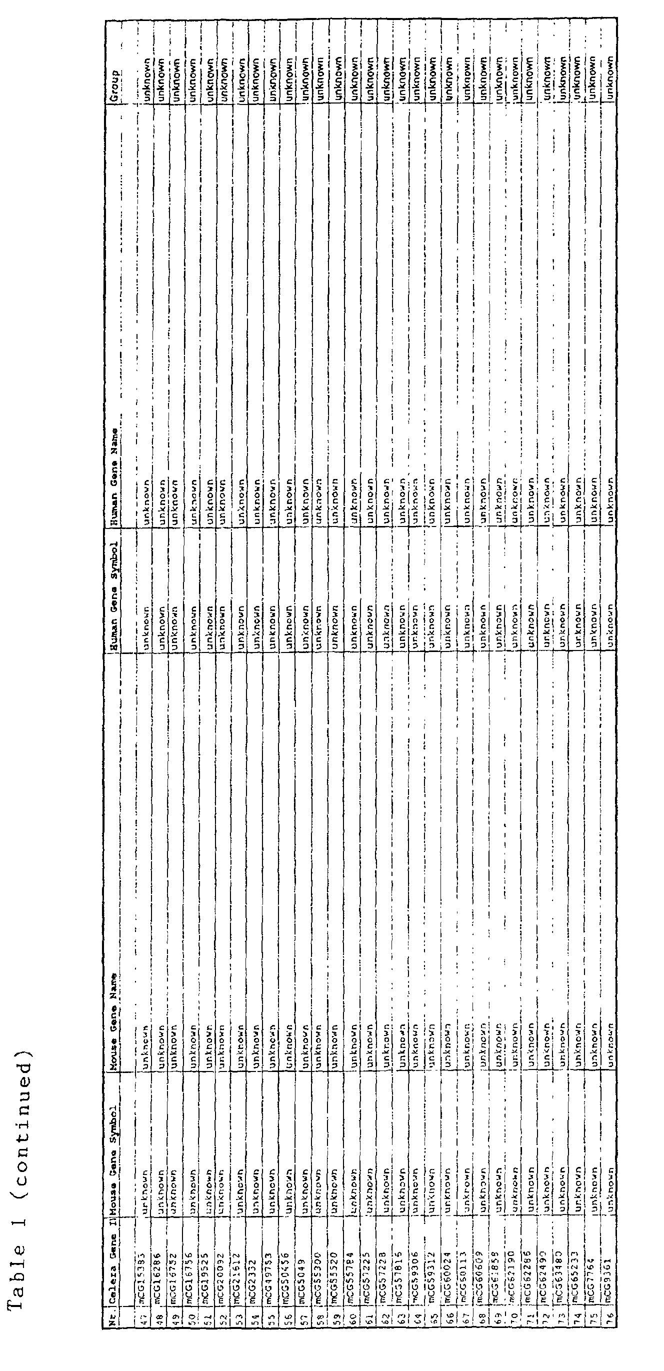

The first object of the present invention to provide novel genes involved in tumor development for use in the design of tumor specific therapies is thus achieved by using the human homologues of the murine genes of Table 1 to develop inhibitors directed against these genes and/or their expression products and to use these inhibitors for the preparation of pharmaceutical compositions for the treatment of cancer.

The term "human homologue" as used herein should be interpreted as a human gene having the same function as the gene identified in mouse.

In one embodiment of the present invention, the inhibitors are antibodies and/or antibody derivatives directed against the expression products of the genes listed in Table 1. Such antibodies and/or antibody derivatives such as scFv, Fab, chimeric, bifunctional and other antibody-derived molecules can be obtained using standard techniques generally known to the person skilled in the art. Therapeutic antibodies are useful against gene expression products located on the cellular membrane. Antibodies may influence the function of their target proteins by for example steric hindrance or blocking at least one of the functional domains of those proteins. In addition, antibodies may be used for deliverance of at least one toxic compound linked thereto to a tumor cell.

In a second embodiment of the present invention, the inhibitor is a small molecule capable of interfering with the function of the protein encoded by the gene involved in tumor development. In addition, small molecules can be used for deliverance of at least one linked toxic compound to a tumor cell.

Small molecule inhibitors are usually chemical entities that can be obtained by screening of already existing libraries of compounds and/or by designing compounds based on the structure of the protein encoded by a gene involved in tumor development. Briefly, the structure of at least a fragment of the protein is determined by either Nuclear Magnetic Resonance or X-ray crystallography. Based on this structure, a screening of compounds can be performed. The selected compounds are synthesized using medicinal and/or combinatorial chemistry and thereafter analyzed for their inhibitory effect on the protein in vitro and in vivo. This step can be repeated until a compound is selected with the desired inhibitory effect. After optimization of the compound, its toxicity profile and efficacy as cancer therapeutic is tested in vivo using appropriate animal model systems.

The expression level of a gene can either be decreased or increased during tumor development. Naturally, inhibitors are used when the expression levels are elevated.

On a different level of inhibition nucleic acids can be used to block the production of proteins by destroying the mRNA transcribed from the gene. This can be achieved by antisense drugs or by RNA interference (RNAi). By acting at this early stage in the disease process, these drugs prevent the production of a disease-causing protein. The present invention relates to antisense drugs, such as antisense RNA and antisense oligodeoxynucleotides, directed against the genes listed in Table 1. Each antisense drug binds to a specific sequence of nucleotides in its mRNA target to inhibit production of the protein encoded by the target mRNA. The invention furthermore relates to RNAi molecules. RNAi refers to the introduction of homologous double stranded RNA to specifically target the transcription product of a gene, resulting in a null or hypomorphic phenotype. RNA interference requires an initiation step and an effector step. In the first step, input double-stranded (ds) RNA is processed into 21-23-nucleotide 'guide sequences'. These may be single- or double-stranded. The guide RNAs are incorporated into a nuclease complex, called the RNA-induced silencing complex (RISC), which acts in the second effector step to destroy mRNAs that are recognized by the guide RNAs through base-pairing interactions. RNAi molecules are thus double stranded RNAs (dsRNAs) that are very potent in silencing the expression of the target gene. The invention provides dsRNAs complementary to the genes listed in Table 1.

The invention relates further to gene therapy, in which the genes listed in Table 1 are used for the design of dominant-negative forms of these genes which inhibit the function of their wild-type counterparts following their directed expression in a cancer cell.

Another object of the present invention is to provide a pharmaceutical composition comprising the inhibitors according to the present invention as active ingredient for the treatment of cancer. The composition can further comprise at least one pharmaceutical acceptable additive like for example a carrier, an emulsifier, or a conservative.

In addition, it is the object of the present invention to provide a method for treatment of cancer patients which method comprises the administration of the pharmaceutical composition according to the invention to cancer patients.

The invention will be further illustrated in the examples that follow and which are not given to limit the invention. Examples 1 and 2 describe how the genes of Table 1 were identified. Example 3 describes the development of inhibitors of the genes and their encoded products.

### EXAMPLES

### EXAMPLE 1

### Identification of genes involved in tumor development using EµMyc and EµMyc; Pim1⁻^{/}⁻; Pim2⁻^{/}⁻ mice

### INTRODUCTION

Retroviral insertions in the genome can transform host cells by activation of proto-oncogenes or inactivation of tumor suppressor genes. Retroviral insertions near such genes are instrumental in the clonal outgrowth of the incipient tumor cell. A full-blown tumor then results from multiple rounds of retroviral insertional mutagenesis in which proviral insertions mark genes collaborating in stepwise tumor progression. To modify the sensitivity of the retroviral screen, different genetic backgrounds can be used to identify oncogenes hardly or not found in retroviral screens using "wild type" background.
On the basis of strong cooperation between *c-Myc* and *Pim* in tumor development, genes acting downstream of, or parallel to *Pim* are likely to be selected for in tumors originating from mice deficient for *Pim* but expressing high levels of *Myc.*

### MATERIALS AND METHODS

### Mice and M-MuLV infection

The *EµMyc* mice were bred with *Pim1* deficient *Pim1neo59* mice and *Pim2* deficient *Pim2K180* mice to generate *EµMyc; Pim1*^{*-*}^{/}^{*-*}*, EµMyc; Pim2*^{*-*}^{/}^{*-*} and *EµMyc; Pim1*^{*-*}^{/}^{*-*}*; Pim2*^{-/-} mice. Neonates were infected with 1.10 infectious units of M-MuLV. Moribund mice were sacrificed and lymphomas were isolated.

### Southern blot analysis of common insertion sites (CISs)

Tumor DNA was isolated. Genomic tumor DNA (10 µg) was restricted with the appropriate enzyme, separated on a 0.7 % agarose gel and subsequently transferred to Hybond-N membranes (Amersham). The number of proviral insertions and the insertions into the known *CIS Pim1, Pim2, Bmi1* and *Gfi1* were analyzed. Genomic fragments, free of repetitive sequences, flanking the proviruses and hybridizing to a CIS were used as probes to analyze the frequency at which these loci were inserted by a provirus.

### Isolation of the proviral insertion sites

### 1. Ligation

Tumor DNA (3 µg) was restricted with BstYI (New England Biolabs) after which the enzyme was inactivated. The splinkerette adaptor was generated by annealing the splinkerette oligos, HMSpAA: 5' cgaagagtaaccgttgctaggagagaccgtggtgaatgagactggtgtcgacactagtgg 3' and HMSpBB: 5' gatccactagtgtgacacagtctctaatttttttttttaaaaaaa 3'. Both oligos contain modifications of a splinkerette. The oligos (150 pmol each) were denatured at 95°C for 3' and subsequently cooled to room temperature at a rate of 1°C per 15" using a thermocycler (PTC100, Perkin Elmer). 600 ng of genomic tumor DNA restricted with BstYI was ligated to the splinkerette oligo (molar ratio 1:10) with 4 U T4 DNA ligase (Roche Diagnostics) in a final volume of 40µl. To avoid amplification of the internal 3' M-MuLV fragment, the ligated fragments were restricted with 10 U of EcoRV in a total volume of 100 µl. Ligation mixtures were desalted in a Microcon YM-30 (Amicon BioSeparations) according to the manufacturer.

### 2. PCR Amplification

M-MuLV flanking sequences were amplified with a radioactive LTR-specific primer, AB949
(5' gctagcttgccaaactcaggtgg 3'), and a splinkerette-primer, HMSp1 (5' cgaagagtaacgttgctaggagagacc 3'). Primer AB949 (10 pmol) was radioactively labeled with γ-³²P ATP (3 µCu) using T4 PNK (0.2U) (Roche Diagnostics). The 50 µl PCR mixture contained 150 ng ligated tumor DNA, 10 pmol primer (each), 300 nmol dNTPs, 1 U PfuITurbo™ and 1X PfuITurbo™ buffer (Stratagene). The hot start PCR conditions were 3' 94°C, 2 cycles 15" 94°C, 30" 68°C, 3' 30" 72°C, 27 cycles 15" 94°C, 30" 66°C, 3' 30" 72°C, and 5' 72°C. Radioactive PCR fragments were concentrated using a microcon-30 (Amicon BioSeparations) and subsequently separated on a 3.5% denaturing polyacrylamide gel. The gels were dried onto 3MM Wattman paper and exposed O/N to X-Omat AR films (Kodak). Amplified fragments were excised from the gel and boiled for 30' in 100 µl TE. 1 µl of the DNA solution was used for a nested amplification with a ³²p labeled virus specific primer HM001 (5' gccaaacctacaggtggggtcttt 3') and a non-radioactive splinkerette-specific primer HMSp2
(5' gtggctgaatgagactggtgtcgac3'). The nested PCR was performed with 5 pmol of primers (each), 200 nM dNTPs (each), 1.75 mM Mg, 1 U Taq polymerase (Gibco BRL), 1X PCR buffer (Gibco BRL) in a final volume of 20 µl. The PCR conditions were 15" 94°C, 30" 60°C, 3' 72°C for 25 cycles (fragments < 400bps) or for 28 cycles (fragments >400bps). The re-amplified fragments were separated on a 3.5% denaturing polyacrylamide gel and isolated as described above. 1 µl of the amplified fragments were again re-amplified in a non-radioactive PCR of 25 cycles under the conditions as described for the radioactive nested PCR.

### 3. Sequence analysis

The nested PCR mixture was treated with 0.5 U exonuclease and 0.5 U shrimp alkaline phoshatase according to the manufacturer (Amersham). About 25 ng of the PCR product was used in the sequence reaction containing BigDye terminator mix (Perkin Elmer) and primer HM001. In addition, HMSp2 was used as primer for sequencing of the amplified fragments larger than 500 bps. Automated sequencing was performed on an ABI 377 (Perkin Elmer). The sequences were processed with Sequencher 3.1.1™ and blasted against the annotated mouse genomic database at Celera using the Celera Discovery System™.

### RESULTS

### Identification of CISs and candidate genes

471 provirus flanks were sequenced from 38 *EµMyc; Pim1*^{*-*}^{/}^{*-*}*; Pim2*^{-/-} and 18 control *EµMyc* lymphomas. This number corresponds to approximately 60% of all retroviral insertions in these tumors. 47 loci showed proviral integrations in more than one tumor and were therefore designated as common insertion sites (CISs). Based on sequence comparisons with the Celera annotated mouse genomic database, the gene located nearby the CIS was identified. Of these genes, the ones that were so far not described to be involved in tumor development are listed in Table 1 combined with the novel cancer genes from Example 2.

### DISCUSSION

Although proviral integrations occur randomly, they may affect the expression or function of nearby genes. If a gene is affected in two or more independent tumors, this indicates that these integrations provide a selective advantage and therefore contribute to tumor development. Multiple of these common insertion sites were identified of which a large number are demonstrated for the first time to play a role in cancer. Importantly, several of the other genes identified are well-known cancer genes validating the approach. This example shows that the pursued strategy can be successfully used to identify novel genes that are involved in tumor development.

### EXAMPLE 2

### Identification of genes involved in tumor development using Cdkn2a-deficient mice

### INTRODUCTION

To identify genes that cooperate with the combined loss of p16^{1nk4a} and p19^{Arf}, encoded by the *Cdkn2a* locus, in tumorigenesis, neonatal mice deficient for the second exon of *Cdkn2a* were infected with Moloney murine leukemia virus (MoMLV). This retrovirus induces tumors by insertional activation of proto-oncogenes or, though inherently more rarely, through inactivation of tumor suppressor genes.

### MATERIALS AND METHODS

### Mo-MLV tumorigenesis

*Cdkn2a* -/- mice as well as +/- and +/+ littermates were infected intraperitoneally with 10⁵ infectious units of Mo-MLV within 72 hours of birth. Diseased mice were euthanized, necropsied, and then *Cdkn2a*-genotyped post-mortem by PCR. Most of each tumor was frozen for genetic studies. Fragments of tumors were fixed in either 10% formalin or Bouin's fixative, paraffin embedded, sectioned, and stained with hematoxylin and eosin. Lymphomas were analyzed by Southern blotting to assess T-cell receptor and immunoglobulin gene rearrangements. Cell surface markers for hematopoietic lineages were assessed by immunohistochemistry. Histiocytic sarcoma transplantation into immunodeficient mice was accomplished by subcutaneous injection near the scapulae of BALB/c SCID mice of approximately 4.5 X 10⁵ disaggregated cells from macroscopically visible histiocytic liver nodules in virally infected *Cdkn2a -*/*-* mice. Tumors appearing in the livers of recipient mice histologically resembled those in the donors. They were confirmed to be of donor origin by PCR genotyping for the exon 2-deleted *Cdkn2a* allele.

### Retrieval and analysis of Mo-MLV integration site sequences

463 viral insertion sites were amplified using splinkerette-aided amplification procedures. 284 insertion site sequences were retrieved using inverse PCR on *Sac*II-digested tumor DNA. The MoMLV-specific primers used for the nested inverse PCR were 5' ATCGGACAGACACAGATAAGTT 3', 5' GCCAAACCTACAGGTGGGGTCTTT 3', 5' AAACCTGTGATGCCTGACCAGT 3', 5' GCAACTGAGACCTGCAAAGCTTGT 3'. Amplification products were purified from agarose gels and subjected to direct automated sequencing. For candidate gene identification, insertion site sequences were filtered for the presence of repetitive DNA and homology searches using BLASTn were performed in GenBank databases as well as in the mouse genome sequence (Celera Genomics). An unambiguous match was defined as having 1 homology region only and a BLASTn probability value of 10²⁵ or less. While CISs traditionally have been demonstrated using Southern blotting, the presence of the complete mouse genome allows for *in silico* CIS determination.
Following the statistical analysis retroviral common insertion sites were defined as 2 or more integrations within 26 kb or 3 insertions within 300kb. For a set of 500 insertions, these windows give a tolerable statistically calculated background of ~2.5 CISs occurring at random. When flanking a gene, the accepted distance between insertions was set to 100kb. While the functionable distance between viral insertions and candidate oncogenes is known to differ between loci, the statistical threshold set here is in accordance with viral integration patterns surrounding previously characterized common insertion sites.

### RESULTS

From a panel of 104 lymphoid (55%) and myeloid (45%) tumors, a total of 747 unique MoMLV integration sites were isolated and directly sequenced using a combination of inverse PCR and splinkerette-aided insertion site amplification. Homology searches in GenBank and in the mouse genome database (Celera Genomics, Rockville, MD) unambiguously mapped 565 viral insertions. 172 of the sequences were clustered in 46 common insertion sites (CISs). Using the Celera annotated mouse genomic database, the genes located nearby the CISs were identified. Of these genes, the ones that were so far not described to be involved in tumor development are listed in Table 1 combined with the novel cancer genes from Example 1.

### DISCUSSION

Although proviral integrations occur randomly, they may affect the expression or function of nearby genes. If a gene is affected in two or more independent tumors, this indicates that these integrations provide a selective advantage and therefore contribute to tumor development. Multiple of these common insertion sites were identified of which a large number are demonstrated for the first time to play a role in cancer. Importantly, several of the other genes identified are well-known cancer genes validating the approach. This example shows that the pursued strategy can be successfully used to identify novel genes that are involved in tumor development.

### EXAMPLE 3

### Preparation of inhibitors of the expression products of genes involved in cancer from Examples 1 and 2

### Confirmation of the involvement of the identified genes in primary human tumors

The expression of the described genes, that were originally identified by genome-wide functional screens involving retroviral insertional tagging in mouse models (see Examples 1 and 2), is determined in a panel of different human tumor samples using microarray analysis. From an extensive set of primary human tumors of various organs, RNA is prepared using standard laboratory techniques to investigate the expression of the described genes in these samples relative to their expression in normal, unaffected tissues from the same origin by using microarrays on which these genes, or parts thereof, are spotted. Microarray analysis allows rapid screening of a large set of genes in a single experiment (DeRisi *et al.* Use of a cDNA microarray to analyse gene expression patterns in human cancer. Nat Genet 14:457-60, 1996; Lockhart *et al.* Expression monitoring by hybridization to high-density oligonucleotide arrays. Nat Biotechnol 14:1649, 1996). Genes that are differentially expressed in tumor and normal tissues as determined by microarray analysis are further examined by other techniques such as RT-PCR, Northern blot analysis, and, if gene-specific antibodies are available, Western blot analysis. Confirmation of differential expression of these genes demonstrates their involvement in human tumors. These findings are further substantiated by similar experiments using a panel of human tumor cell lines.

### Functional importance of the identified genes for human cancer

Subsequently, the functional importance of the differentially expressed genes for tumor cells is determined by over-expression as well as by inhibition of the expression of these genes. Selected human tumor cell lines are transfected either with plasmids encoding cDNA of the genes or with plasmids encoding RNA interference probes for the genes. RNA interference is a recently developed technique that involves introduction of double-stranded oligonucleotides designed to block expression of a specific gene (see Elbashir *et al.* Duplexes of 21-nucleotide RNAs mediate RNA interference in cultured mammalian cells. Nature 411:494-8, 2001; Brummelkamp *et al.* A system for stable expression of short interfering RNAs in mammalian cells. Science 296:550-3, 2002). Using standard laboratory techniques and assays, the transfected cell lines are extensively checked for altered phenotypes that are relevant for the tumor cells, e.g. cell cycle status, proliferation, adhesion, apoptosis, invasive abilities, etc. These experiments demonstrate the functional importance of the identified genes for human cancer.

### Structure-function analysis of selected targets

Genes that are shown to be both differentially expressed and functionally important for human tumors are selected for structure-function analysis. Deletion and point mutation mutants of these genes are constructed and tested for their functional competence compared with wild-type genes (according to Ibanez *et al.* Structural and functional domains of the myb oncogene: requirements for nuclear transport, myeloid transformation, and colony formation. J Virol 62:1981-8, 1988; Rebay et *al.* Specific truncations of Drosophila Notch define dominant activated and dominant negative forms of the receptor. Cell 74:319-29, 1993). These studies lead to the identification of functionally critical domains as well as to dominant-negative variants, i.e. mutant genes that suppress the function of their endogenous counterparts (e.g. Kashles *et al.* A dominant negative mutation suppresses the function of normal epidermal growth factor receptors by heterodimerization. Mol Cell Biol 11:1454-63, 1991). These dominant-negatives provide additional proof for the functional importance of the genes and give insight into which therapeutic approaches can be pursued to interfere with their function. Furthermore, the cellular localization of the proteins encoded by the genes is determined by immunofluorescence using standard laboratory techniques. If the proteins are expressed on the cell-surface, this allows for the development of antibody-based inhibitors.

### Development of antibody-based inhibitors

Differentially expressed genes that encode membrane-bound proteins are selected as targets for conventional antibody-based therapies. Antibodies are generated against functionally relevant domains of the proteins and subsequently screened for their ability to interfere with the target's function using standard techniques and assays (Schwartzberg. Clinical experience with edrecolomoab: a monoclonal antibody therapy for colorectal carcinoma. Crit Rev Oncol Hematol 40:17-24, 2001; Herbst *et al.* Monoclonal antibodies to target epidermal growth factor receptor-positive tumors: a new paradigm for cancer therapy. Cancer 94:1593-611, 2002).

### Development of small molecule inhibitors

Differentially expressed genes that do not encode membrane-bound proteins are selected as targets for the development of small molecule inhibitors. To identify putative binding sites or pockets for small molecules on the surface of the target proteins, the three-dimensional structure of those targets are determined by standard crystallization techniques (de Vos *et al.* Three-dimensional structure of an oncogene protein: catalytic domain of human c-H-ras p21. Science 239:888-93, 1988; Williams *et al.* Crystal structure of the BRCT repeat region from the breast cancer-associated protein BRCA1. Nat Struct Biol 8:838-42, 2001). Additional mutational analysis is performed as mentioned above to confirm the functional importance of the identified binding sites. Subsequently, Cerius2 (Molecular Simulations Inc., San Diego, CA, USA) and Ludi/ACD (Accelrys Inc., San Diego, CA, USA) software is used for virtual screening of small molecule libraries (Bohm. The computer program Ludi: A new method for the de novo design of enzyme inhibitors. J Comp Aided Molec Design 6:61-78, 1992). The compounds identified as potential binders by these programs are synthesized by combinatorial and medicinal chemistry and screened for binding affinity to the targets as well as for their inhibitory capacities of the target protein's function by standard *in vitro* and *in vivo* assays. In addition to the rational development of novel small molecules, existing small molecule compound libraries are screened using these assays to generate lead compounds. Lead compounds identified are subsequently co-crystallized with the target to obtain information on how the binding of the small molecule can be improved (Zeslawska *et al.* Crystals of the urokinase type plasminogen activator variant beta(c)-uPAin complex with small molecule inhibitors open the way towards structure-based drug design. J Mol Biol 301:465-75, 2000). Based on these findings, novel compounds are designed, synthesized, tested, and co-crystallized. This optimization process is repeated for several rounds leading to the development of a high-affinity compound of the invention that successfully inhibits the function of its target protein. Finally, the toxicity of the compound is tested using standard assays (commercially available service via MDS Pharma Services, Montreal, Quebec, Canada) after which it is screened in an animal model system.

### Development of antisense molecule inhibitors

These inhibitors are either antisense RNA or antisense oligodeoxynucleotides (antisense ODNs) and are prepared synthetically or by means of recombinant DNA techniques. Both methods are well within the reach of the person skilled in the art. ODNs are smaller than complete antisense RNAs and have therefore the advantage that they can more easily enter the target cell. In order to avoid their digestion by DNAse, ODNs but also antisense RNAs are chemically modified. For targeting to the desired target cells, the molecules are linked to ligands of receptors found on the target cells or to antibodies directed against molecules on the surface of the target cells.

### Development of RNAi molecule inhibitors

Double-stranded RNA corresponding to a particular gene is a powerful suppressant of that gene. The ability of dsRNA to suppress the expression of a gene corresponding to its own sequence is also called post-transcriptional gene silencing or PTGS. The only RNA molecules normally found in the cytoplasm of a cell are molecules of single-stranded mRNA. If the cell finds molecules of double-stranded RNA, dsRNA, it uses an enzyme to cut them into fragments containing 21-25 base pairs (about 2 turns of a double helix). The two strands of each fragment then separate enough to expose the antisense strand so that it can bind to the complementary sense sequence on a molecule of mRNA. This triggers cutting the mRNA in that region thus destroying its ability to be translated into a polypeptide. Introducing dsRNA corresponding to a particular gene will knock out the cell's endogenous expression of that gene. This can be done in particular tissues at a chosen time. A possible disadvantage of simply introducing dsRNA fragments into a cell is that gene expression is only temporarily reduced. However, a more permanent solution is provided by introducing into the cells a DNA vector that can continuously synthesize a dsRNA corresponding to the gene to be suppressed. RNAi molecules are prepared by methods well known to the person skilled in the art.

### Efficacy of antibody-based and small molecule inhibitors in animal model systems

The efficacy of both the antibody-based and small molecule inhibitors are tested in an appropriate animal model system before entry of these inhibitors into clinical development (e.g. Brekken *et al.* Selective inhibition of vascular endothelial growth factor (VEGF) receptor 2 (KDR/Flk-1) activity by a monoclonal anti-VEGF antibody blocks tumor growth in mice. Cancer Res 60:5117-24, 2000; Wilkinson *et al.* Antibody targeting studies in a transgenic murine model of spontaneous colorectal tumors. Proc Natl Acad Sci USA 98:10256-60, 2001; Laird et al. SU6668 inhibits Flk-1/KDR and PDGFRbeta in vivo, resulting in rapid apoptosis of tumor vasculature and tumor regression in mice. FASEB J 16:681-90, 2002).

## Claims

1. Use of inhibitors of the expressed proteins of the murine genes and/or their human homologues listed in Table 1 for the preparation of a therapeutical composition for the treatment of cancer, in particular for the treatment of solid tumors of lung, colon, breast, prostate, ovarian, pancreas and leukemia.

2. Use as claimed in claim 1, wherein the inhibitors are antibodies or derivatives thereof directed against the expression products of the genes that are expressed on the cell membrane.

3. Use as claimed in claim 2, wherein the derivatives are selected from the group consisting of scFv fragments, Fab fragments, chimeric antibodies, bifunctional antibodies, and other antibody-derived molecules.

4. Use as claimed in claim 1, wherein the inhibitors are small molecules interfering with the biological activity of the protein expressed by the gene.

5. Use of inhibitors of the mRNA transcripts of the genes listed in Table 1 for the preparation of a therapeutical composition for the treatment of cancer.

6. Use as claimed in claim 5, wherein the inhibitors are antisense molecules, in particular antisense RNA or antisense oligodeoxynucleotides.

7. Use as claimed in claim 5, wherein the inhibitors are double stranded RNA molecules for RNA interference.

8. Use as claimed in claim 1 and claim 5, wherein the treatment comprises gene therapy.

9. Use as claimed in any one of the claims 1-8, wherein the therapeutical composition is for treatment of inflammatory diseases.

10. Inhibitor compound directed against the expressed proteins of a murine gene and/or its human homologue listed in Table 1 for use in the treatment of cancer.

11. Inhibitor compound as claimed in claim 10, which is an antibody or derivatives thereof directed against the expression products of a gene that is expressed on the cell membrane.

12. Inhibitor compound as claimed in claim 11, wherein the derivative is selected from the group consisting of scFv fragments, Fab fragments, chimeric antibodies, bifunctional antibodies, or other antibody-derived molecules.

13. Inhibitor compound as claimed in claim 10, which is a small molecule interfering with the biological activity of the protein expressed by the gene.

14. Inhibitor compound directed against the transcription product (mRNA) of a murine gene and/or its human homologue listed in Table 1 for use in the treatment of cancer.

15. Inhibitor compound as claimed in claim 14, which is an antisense molecule, in particular an antisense RNA or an antisense oligodeoxynucleotide.

16. Inhibitor compound as claimed in claim 15, which is a double stranded RNA molecule for RNA interference.

17. Therapeutical composition for the treatment of cancers in which one or more of the murine genes and/or their human homologues listed in Table 1 are involved, comprising a suitable excipient, carrier or diluent and one or more inhibitor compounds as claimed in claims 10-16.

18. Compositions as claimed in claim 17, wherein the cancer is a solid tumor of e.g. lung, colon, breast, prostate, ovarian, pancreas and leukemia.

19. Method for the development of therapeutic inhibitor compounds as claimed in claims 10-16, which method comprises the steps:
a) identification of genes involved in cancer, in particular by using retroviral insertional tagging, optionally in a specific genetic background;
b) validation of one or more of the identified genes as potential target gene(s) for the therapeutic compound by one or more of the following methods:
c) confirmation of the identified gene by Northern Blot analysis in cancer cell-lines;
d) determination of the expression profile of the identified gene in tumors and normal tissue;
e) determination of the functional importance of the identified genes for cancer;
f) production of the expression product of the gene; and
g) use of the expression product of the gene for the production or design of a therapeutic compound.

20. Method as claimed in claim 19, wherein the gene identified in step a) is selected from the murine genes and/or their human homologues listed in Table 1.
